# EUROPEAN PATENT APPLICATION

(11) **EP 1 767 150 A2**
(43) Date of publication of application: **28.03.2007**
(21) Application number: 06254938.1
(22) Date of filing: 25.09.2006
(51) Int. Cl.: A61B 5/15

(54) **Device for promoting bodily fluid expression**

(30) Priority: 26.09.2005 US 236119
(71) Applicant: Lifescan Scotland Ltd, Inverness-shire IV2 3ED (GB)
(72) Inventor: Thomson, Anne, Strathpepper Ross-shire IV14 9AT (GB); Alvarez-Icaza, Manuel, Inverness Inverness-shire IV2 4FL (GB); Gordon, Wilma, Inverness Inverness-shire IV2 4EH (GB)
(74) Representative: Tunstall, Christopher Stephen

(57) **Abstract**

A device (100) for promoting bodily fluid expression from a target site includes urging a target site of a user's body toward a device for promoting expression of bodily fluid from the target site. The device also includes means (104) for sequentially increasing an area of contact and applied pressure between a pressure application mechanism (104) of the device and the user's body in the vicinity of the target site, and in a direction toward the target site, thereby urging bodily fluid toward the target site. Thereafter, the target site can be lanced.

## Description

### BACKGROUND OF THE INVENTION

1. Field of the Invention

The present invention relates, in general, to medical devices and associated methods and, in particular, to devices and methods for promoting bodily fluid expression from a target site.

2. Description of the Related Art

A variety of medical conditions, such as diabetes, call for the monitoring of an analyte concentration (e.g., glucose concentration) in a blood, interstitial fluid or other bodily fluid sample. Typically, such monitoring requires the expression of a bodily fluid sample from a target site (e.g., a dermal tissue target site on a user's finger).

The expression (also referred to as "extraction" as circumstances dictate) of a blood sample from a dermal tissue target site on a user's fingertip generally involves lancing the dermal tissue target site and applying pressure in the vicinity of the lanced site to express the blood sample. The user can apply the pressure by "milking" the lanced finger using their opposing hand. Such milking typically involves applying pressure circumferentially around the first knuckle using the thumb and finger(s) of the opposite hand, thereby restricting the flow of blood into and away from the lanced target site. The area of applied pressure is then moved towards the lanced target site (namely the lanced fingertip) by sliding the thumb and finger(s) toward the lanced fingertip, thus causing the lanced fingertip to engorge with blood and promoting the expression a blood sample from the lanced target site.

### BRIEF DESCRIPTION OF THE DRAWINGS

A better understanding of the features and advantages of the present invention will be obtained by reference to the following detailed description that sets forth illustrative embodiments, in which the principles of the invention are utilized, and the accompanying drawings in which like numerals represent like elements, of which:

FIG. 1 is a simplified perspective view of a device for promoting the expression of bodily fluid from a target site according to an exemplary embodiment of the present invention;

FIG. 2 is a simplified cross-sectional view depicting alignment and movement of user's finger with respect to a portion of the device of FIG. 1;

FIG. 3 is a simplified cross-sectional view depicting movement and forces within a user's finger as the user's finger is urged against (i.e., engages with) a portion of the device of FIG. 1;

FIG. 4 is a simplified perspective view of an analyte monitoring system with a device for promoting bodily fluid expression from a target site according to an exemplary embodiment of the present invention;

FIG. 5 is a simplified perspective view of the analyte monitoring system of FIG. 4 in use (wherein dashed lines indicate certain components hidden from view in the perspective of FIG. 5);

FIG. 6 is a simplified perspective view of an analyte monitoring system with a device for promoting bodily fluid expression from a target site according to another exemplary embodiment of the present invention in use (wherein dashed lines indicate certain components hidden from view in the perspective of FIG. 6);

FIG. 7 is a simplified side and cross-sectional view of a device for promoting expression of bodily fluid from a target site according to another exemplary embodiment of the present invention depicting a user's finger being inserted into the device in the direction of arrow D;

FIG. 8 is a is a simplified side and cross-sectional view of the device of FIG. 8 depicting a user's finger being urged toward a pressure application mechanism of the device in the direction of arrow E;

FIG. 9 is a simplified side and cross-sectional view of a device of FIG. 8 depicting a user's finger being further urged toward a pressure application mechanism of the device in the direction of arrow G;

FIG. 10 is a simplified perspective view of analyte monitoring system with a device for promoting bodily fluid expression from a target site according to another exemplary embodiment of the present invention; and

FIG. 11 is a flowchart illustrating a sequence of steps in a process for promoting bodily fluid expression from a target site according to an exemplary embodiment of the present invention.

### DETAILED DESCRIPTION OF THE INVENTION

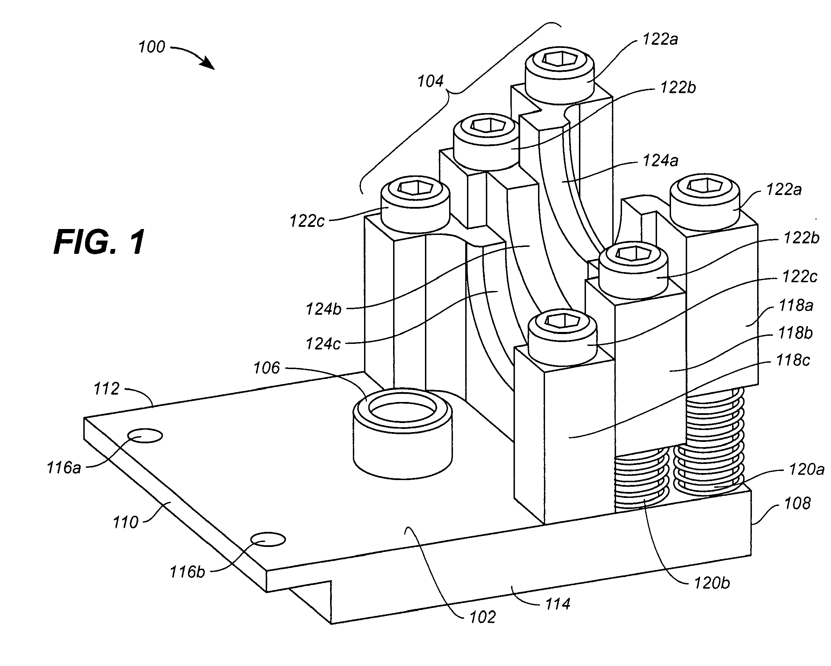

FIG. 1 is a simplified perspective view of a device 100 for promoting the expression of bodily fluid from a target site on a user's body according to an embodiment of the present invention. The embodiment of FIG. 1 (i.e., device 100) is particularly adapted for promoting the expression of blood from a lanced target site on a user's fingertip.

Device 100 includes a base 102, a pressure application mechanism 104 attached to base 102. Base 102 includes a rimmed aperture 106. Pressure application mechanism 104 is configured for urging bodily fluid toward the target site. In addition and as described in detail below, pressure application mechanism 104 is configured for urging the bodily fluid toward the target site by sequentially increasing an area of contact and applied pressure between the pressure application mechanism and the user's body in the vicinity of the target site. Furthermore, the area of contact and applied pressure sequentially increases in a direction toward the target site.

Base 102 further includes a proximal end 108, a distal end 110, first longitudinal side 112, second longitudinal side 114 and openings 116a and 116b therethrough. Base 102 can be formed of any suitable rigid material and serves as a relatively stable foundation for pressure application mechanism 104. If desired, base 102 can be configured, for example, for integration into an analyte monitoring system or lancing device.

Openings 116a and 116b can be employed, for example, to integrate device 100 with other components of an analyte monitoring system via bolts or other suitable fastening techniques. Although, for the purpose of explanation only, two openings are depicted in the base, any suitable number of openings can be employed.

Pressure application mechanism 104 includes ribs 118a, 118b and 118c, springs 120a and 120b operatively interfaced to ribs 118a and 118b, respectively, and a plurality of bolts 122a, 122b, and 122c. Ribs 118a, 118b, and 118c each include a semicircular contact surface 124a, 124b and 124c, respectively, for engaging (i.e., contacting) a user's body in the vicinity of a target site.

Ribs 118a and 118b are attached to base 102 by bolts 122a and 122b, respectively. Moreover, ribs 118a and 118b are moveable along bolts 122a and 122b against the biasing force of springs 120a and 120b, respectively, during use of device 100. Rib 118c is attached to base 102 by bolts 122c and is stationary (i.e., fixed) with respect to base 102.

Ribs 118a, 118b and 118c can be formed of any suitable material including, but not limited to, polystyrene, polycarbonate and polyester. Ribs 118a, 118b and 118c can be entirely rigid or have a relatively soft semicircular contact surfaces (i.e., surfaces 124a, 124b and 124c) for conforming to the contours of a user's finger. The semicircular shape of the contact surfaces provides for pressure to be beneficially applied partially around the circumference of a user's finger as opposed to simply linearly across the user's finger.

Once apprised of the present disclosure, one skilled in the art will recognize that device 100 includes two springs 120a operatively interfaced to rib 118a and two springs 120b operatively interfaced to rib 118b, although only one spring 120a and one spring 120b are visible in the perspective of FIG. 1. Moreover, although device 100 includes three ribs, with ribs 118a and 118b being moveable and rib 118c being stationary (i.e., fixed), the plurality of ribs employed in embodiments of the present invention can be any suitable number.

Rimmed aperture 106 is centered on base 102, generally cylindrical in shape and the aperture thereof provides a passageway for a lancet (not shown) of a dermal tissue lancing mechanism (not shown) to pass therethrough to penetrate dermal tissue. During use of device 100, a user positions a target site on one of the user's fingertips on rimmed aperture 106.

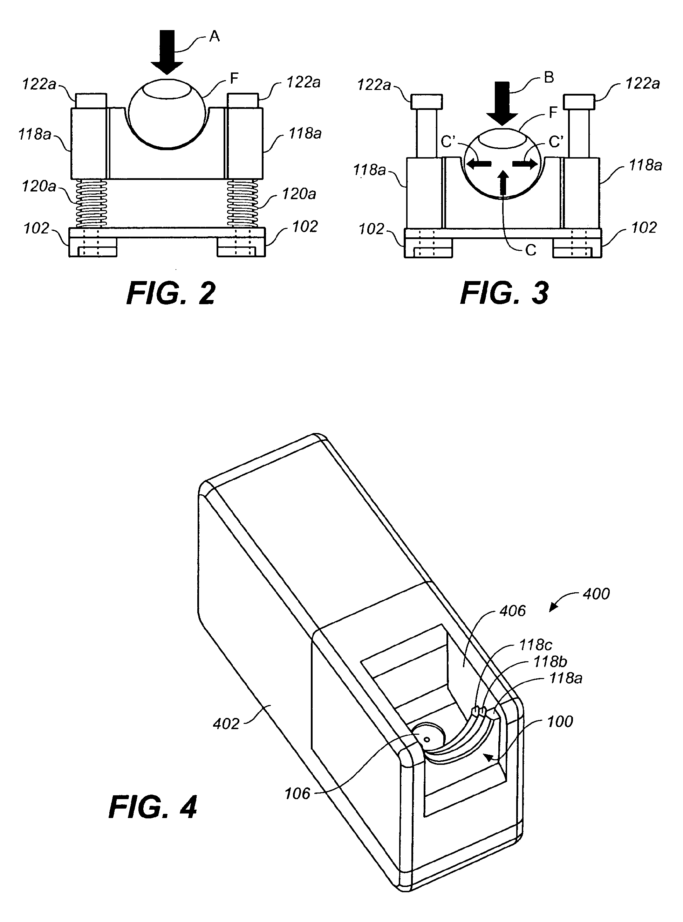

FIGs. 2 and 3 are simplified cross-sectional views depicting movement and pressure within a user's finger F and rib 118a of device 100. In FIG. 2, the open arrow indicates the direction of movement of finger F. In FIG. 3, the open arrow indicates both the direction of movement of finger F and the pressure being applied by finger F on rib 118a. In addition and as described below, the filled arrows of FIG. 3 indicate displacement of finger F tissue. For example, as finger F is pressed against rib 118a, finger tissue is forced upwards as indicated by filled arrow C. As a result of this upward movement of finger tissue, finger tissue is also forced sideways against rib 118a, as indicated by filled arrows C'.

Referring to FIGs. 1-3, during use of device 100, user's finger F is operatively aligned with pressure application mechanism 104 and rimmed aperture 106 and subsequently urged toward contact surfaces 124a, 124b, and 124c and base 102.

Referring to FIG. 2 in particular, user's finger F is urged toward contact surface 124a of rib 118a in the direction of open arrow A in FIG. 2. Once user's finger F engages contact surface 124a, a predetermined pressure (force) indicated by open arrow B (as depicted in FIG. 3) is applied by ringer F to contact surface 124a, resulting in finger tissue displacement indicated by filled arrows C and C'. Rib 118a reacts by transferring the pressure to springs 120a and subsequently moving downward while compressing springs 120a. Such downward movement of rib 118a is halted when rib 118a makes contact with base 102 (see FIG. 3).

One of skill in the art will recognize that springs 120a serve to bias rib 118a and that springs 120b serve to bias rib 118b. However, ribs in embodiments of the present invention can be biased by suitable techniques other than springs, including, for example, techniques that employ elastomeric blocks and techniques that involve forming the ribs themselves at least partially of elastomeric materials.

As rib 118a moves downward towards base 102, contact surface 124a applies an increasing counter-pressure to user's finger F due to the biasing arrangement of springs 120a. The counter-pressure applied by contact surface 124a results in blood (not shown in the FIGs.) being urged toward, and pooling within, the tip of user's finger F. The semicircular contour of contact surface 124a is configured to restrain deformation of user's finger F and, thereby, promote the aforementioned urging and pooling of blood with the tip of user's finger F.

During use of device 100, as user's finger F is urged toward contact surfaces 124a, 124b and 124c, each of these contact surfaces serves to sequentially increase an area of contact and applied pressure between the pressure application mechanism and the user's body in the vicinity of the target site. In other words, the area of contact and applied pressure is first defined by contact surface 124a, then sequentially increases to the sum of the area defined by contact surfaces 124a and 124b and finally sequentially increases to the area defined by contact surfaces 124a, 124b and 124c. The sequential increase in the area of contact and applied pressure therefore occurs in the direction of the target site. For example, the area of contact and applied pressure can sequentially increase from a position below a last knuckle of the user's finger to half way between the last knuckle and the fingertip. The final applied pressure can exert a force in the range from, for example, 15N to 20N.

As the area of contact and applied pressure increases in the direction of the target site (i.e. toward the user's fingertip), it is postulated without being bound that occlusion of capillary blood vessels within the user's finger occurs. As blood is essentially trapped and pressurized between the increasing area of contact and applied pressure and the end the capillary blood vessels at the distal end of the user's fingertip, expression of blood from the fingertip, once lanced, is promoted. As blood capillary vessels run predominantly perpendicular to ribs 118a, 118b and 118 (i.e., along the length of user's finger F), they are occluded by the compressive forces (pressure) that develops between ribs 118a, 118b and 118c and the bone structure of user's finger F.

In the embodiment of FIG. 1, contact surfaces 124a, 124b and 124c are immediately adjacent to one another upon application of a predetermined force to ribs 118a and 118b by a user's finger that results in ribs 118a and 118b contacting base 102. Therefore, in the embodiment of FIG. 1, base 102 serves as a "stop" for ribs 118a and 118b by preventing further downward movement of ribs 118a and 118b once they have contacted base 102.

Due to the sequentially increasing area of contact and applied pressure, the blood within the fingertip is prevented from flowing away from the fingertip and is pressurized within the fingertip. Such pressurization promotes expression of blood from the target site once the target site has been lanced.

Once a user's finger has depressed both rib 118a and 118b such that they are in contact with base 102, the tip of the user's finger is positioned in operative contact with rimmed aperture 106 and, thereby, properly aligned for lancing. Such lancing can be accomplished using any suitable dermal tissue lancing device. Moreover, such a dermal tissue lancing mechanism can be either an independent device or a device that is integrated with an analyte monitoring system (as will be described below with respect to, for example, FIG. 4). Suitable dermal tissue lancing devices are described in, for example, US. Patent No. 6,197,040, which is hereby fully incorporated herein by reference.

The dermal tissue lancing devices can employ a suitable dermal tissue penetration member (e.g., a lancet). The dermal tissue lancing device can be a component of an integrated medical device that also includes a test strip(s), examples of which are described in International Application No. PCT/GB01/05634 (published as WO 02/49507 on June 27, 2002) and U.S. Patent Application No. 10/143,399, both of which are fully incorporated herein by reference.

Once apprised of the present invention, those skilled in the art will recognize that embodiments of the present invention can also be used for promoting the expression of a bodily fluid sample for subsequent testing by a separate analyte monitoring system or integrated within an analyte monitoring system according to embodiments of the present invention described herein.

Pressure application mechanism 104 of device 100 beneficially requires no motorized components to create the counter-pressure that is applied in the vicinity of a target site. In addition, during use of device 100, the target site can be on an underside of a user's fingertip and the area of sequentially increasing area of contact and applied pressure is also on the underside of the user's finger. Applying pressure on the same side of a finger as the target site is believed to enhance the effectiveness of the applied pressure in promoting bodily fluid (e.g., blood) expression, as well as enabling the device to be compact in nature.

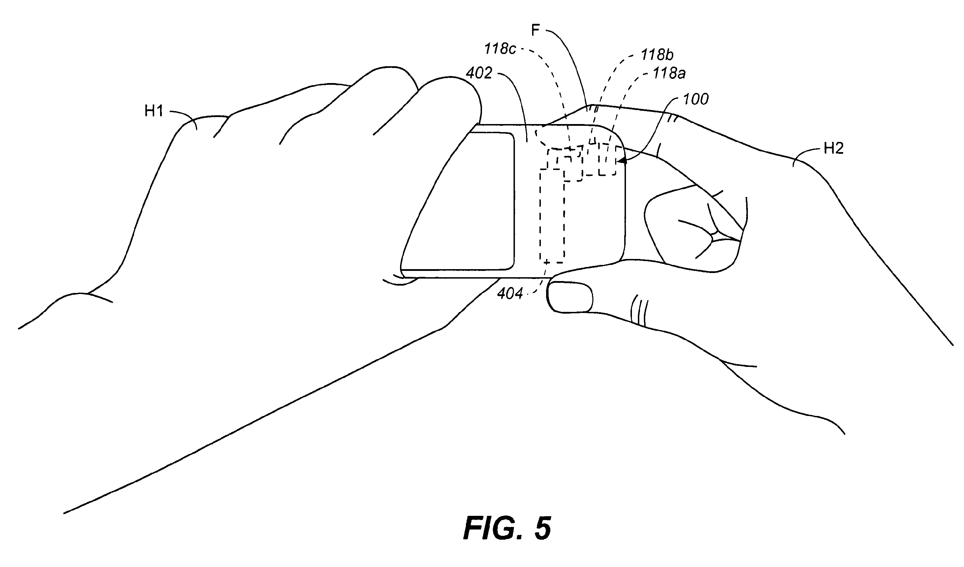

FIG. 4 is a simplified perspective view of an analyte monitoring system 400 with a device for promoting bodily fluid expression from a target site according to an embodiment of the present invention. FIG. 5 is a simplified perspective view of analyte monitoring system 400 in use.

Referring to FIGs. 4 and 5, analyte monitoring system 400 includes a housing 402, a meter (not shown) for the determination of an analyte (e.g., blood glucose) in a bodily fluid sample (such as a blood sample) at least partially contained within housing 402, a dermal tissue lancing device 404 (not shown in FIG. 4 but depicted with dashed lines in FIG. 5) and a device for promoting the expression of a bodily fluid sample from a target site. Any suitable meter for the determination of an analyte in a bodily fluid sample can be employed in analyte monitoring systems according to the present invention including, but not limited to, for example, the meters described in U.S. Patent Nos. 6,489, 133 and 6,335,203, which are hereby incorporated in full by reference.

In the embodiment of FIG. 4, the device for promoting the expression of a bodily fluid sample is device 100 of FIGs. 1, 2 and 3 and, therefore, includes rimmed aperture 106, ribs 118a, 118b, 118c and the remainder of the components of device 100 described above. However, any suitable device for promoting the expression of bodily fluid from a target site according to embodiments of the present invention can be employed in analyte monitoring systems according to the present invention. Such devices include a pressure application mechanism for urging bodily fluid toward the target site, with the pressure application mechanism being configured for urging the bodily fluid by sequentially increasing an area of contact and applied pressure between the pressure application mechanism and the user's body in the vicinity of the target site.

Moreover, the device is attached to housing 402 of monitoring system 400 via, for example, a base of the device. Alternatively, a portion of housing 402 can serve as a base for the device.

Housing 402 includes a recess 406 for containing device 100 and providing access for a user to urge a finger toward, and engage with, device 100 (as shown in FIG. 5). Rimmed aperture 106 is aligned with dermal tissue lancing device 404 (see FIG. 5).

FIG. 5 depicts a manner in which a user can hold analyte monitoring system 400 in one hand (H1) and simultaneously position a finger F of the opposing hand (H2) within indent 406. The user then urges finger F toward device 100 as described above with respect to FIGs. 1, 2 and 3. Once a target site on the user's finger is operatively contacted with rimmed aperture 106 and ribs 118a and 118b have been depressed such that they make contact with base 102, dermal tissue lancing device 404 lances the target site and a bodily fluid sample is expressed from the target site under the applied pressure of device 100. The bodily fluid sample is subsequently analyzed (determined) by the meter.

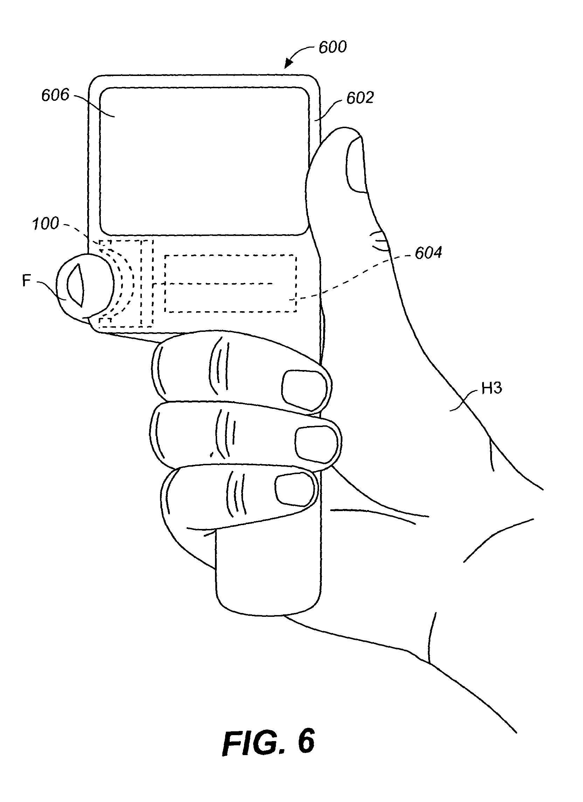

FIG. 6 is a simplified perspective view of an analyte monitoring system 600 that includes a device for promoting bodily fluid expression from a target site according to another embodiment of the present invention. Analyte monitoring system 600 includes a housing 602, a meter (not shown) for the determination of an analyte (e.g., blood glucose) in a bodily fluid sample (such as a blood sample) at least partially contained within housing 602, a dermal tissue lancing device 604 and a device for promoting the expression of a bodily fluid sample from a target site.

In the embodiment of FIG. 6, the device for promoting the expression of a bodily fluid sample is device 100 of FIGs. 1, 2 and 3 and, therefore, includes the components of device 100 described above. Housing 602 of analyte monitoring system 600 includes a display 606 for visually communicating analysis results from the meter contained within housing 602 to a user.

Analyte monitoring system 600 may be beneficially operated by a single hand H3 (i.e., the hand of the finger which is to be lanced as is shown in FIG. 6). Otherwise, the embodiment of FIG. 6 operates in the manner of analyte monitoring system 400 of FIGs. 4 and 5 as described above. During use analyte monitoring system 600, pressure generate by user's finger F is transferred to base 102 (not shown in FIG. 6), subsequently to housing 602, and then to hand H3 as a reaction force. Therefore, in the embodiment of FIG. 6, pressure is applied and a corresponding reaction force absorbed by hand H3. This is accomplished since hand H3 is holding analyte monitoring system 600 in a gripping action with forces exerted between the thumb of hand H3 and the fingers of hand H3. Such a gripping action is beneficially natural and comfortable to a user.

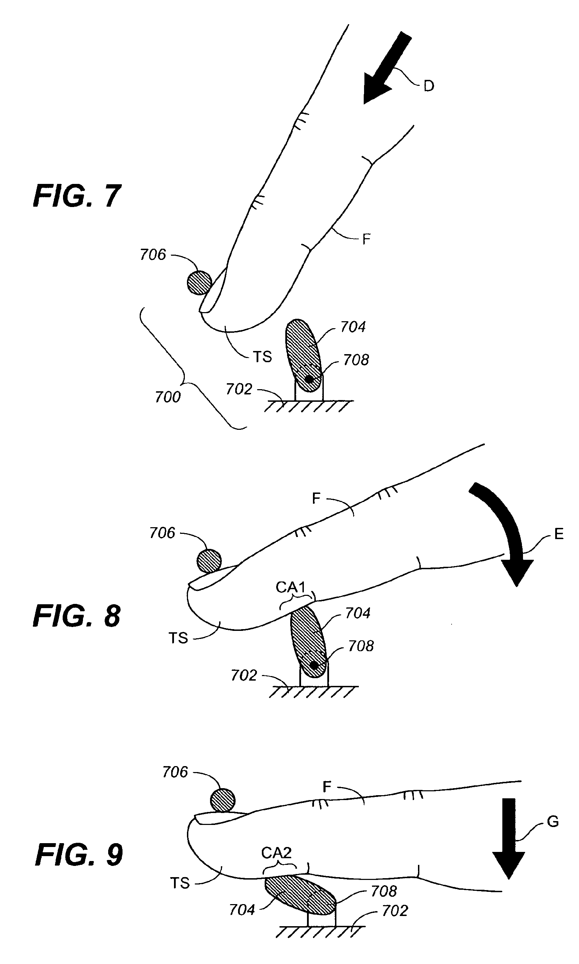

FIG. 7 is a simplified side and cross-sectional view of a device 700 for promoting expression of bodily fluid (e.g., blood) from a target site TS (such as a lanced fingertip target site) according to yet another exemplary embodiment of the present invention depicting a user's finger F being inserted into device 700 in the direction of arrow D. FIGs. 8 and 9 depict user's finger F being urged toward, and engaged with, a pressure application mechanism of the device in the direction of arrows E and G, respectively.

Device 700 includes a base 702 and a pressure application mechanism with a biased cam 704 pivotally attached to base 702 and a stationary leverage bar 706 (shown in cross-section) offset from the biased cam 704 by a distance whereby a portion of the user's body (i.e., finger F of FIG. 7) can be inserted between biased cam 704 and stationary leverage bar 706.

Biased cam 704 includes a biasing spring 708 (for example, a torsional spring). Biasing spring 708 restrains the pivoting of biased cam 704 until the force applied to biased cam 704 (and thus the counter-force applied by biased cam 704 to user's finger F) exceeds a predetermined value. Stationary leverage bar 706 aids in preventing user's finger F from disengaging from biased cam 704 during use of device 700.

The pressure application mechanism of device 700 is configured for urging bodily fluid toward a target site. In addition and as described in detail below, the pressure application mechanism is configured for urging the bodily fluid toward the target site by sequentially increasing an area of contact and applied pressure between the pressure application mechanism and the user's body in the vicinity of the target site.

Referring to FIGs. 8 and 9, when user's finger F is urged downward against biased cam 704, biased cam 704 begins applying a pressure (i.e., a counter-pressure) against user's finger F across area of contact and applied pressure CA1 (see FIG. 8). As user's finger F is further urged toward biased cam 704 (see FIG. 9), the area of contact and applied pressure increases to CA2 due to the biased rotation of biased cam 704 toward the tip of user's finger F. The increase in the area of contact and applied pressure from CA1 to CA2 serves to urge bodily fluid (e.g., blood) into the fingertip of user's finger F and promote the subsequent expression of the bodily fluid from a target site on the fingertip. As biased cam 704 is spring biased, no motorized components are required to produce the applied pressure.

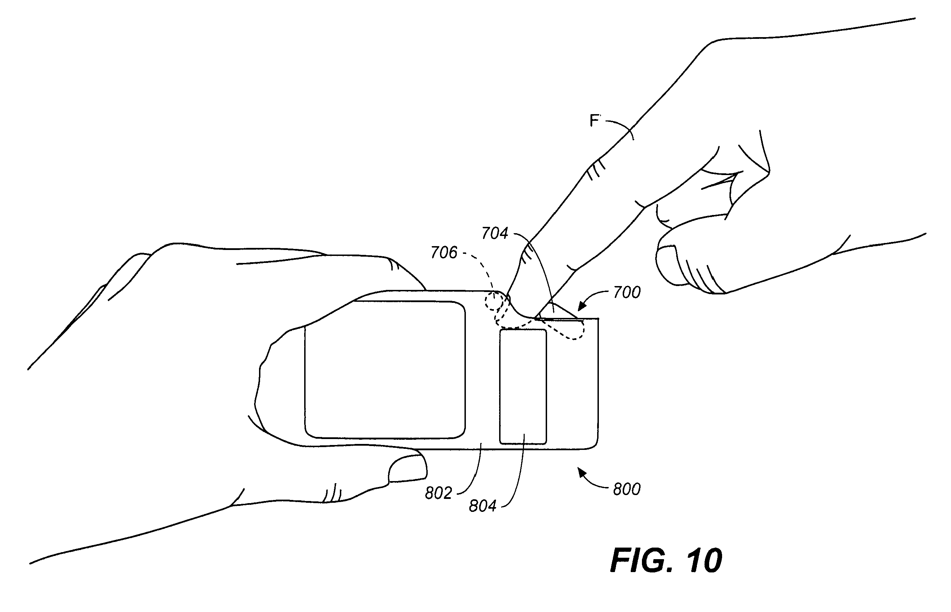

FIG. 10 is a simplified perspective view of another analyte monitoring system 800 with a device for promoting bodily fluid expression from a target site on a user's finger F according to an embodiment of the present invention. Analyte monitoring system 800 includes a housing 802, a meter (not shown) for the determination of an analyte (e.g., blood glucose) in a bodily fluid sample (such as a blood sample) at least partially contained within housing 802, a dermal tissue lancing device 804 and a device for promoting the expression of a bodily fluid sample from a target site.

In the embodiment of FIG. 10, the device for promoting the expression of a bodily fluid sample is device 700 of FIGs. 7, 8 and 9 and, therefore, includes the components of device 700 described above. FIG. 10 depicts user's finger F in approximately the position of FIG. 8.

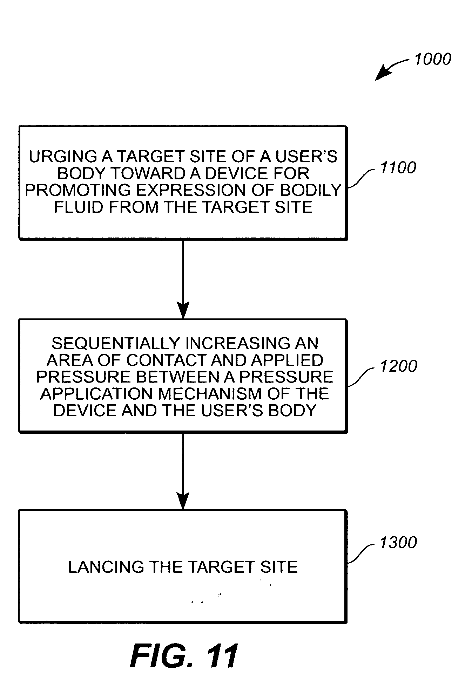

FIG. 11 is a flowchart illustrating a sequence of steps in a process 1000 for promoting bodily fluid expression from a target site according to an exemplary embodiment of the present invention. Process 1000 includes urging a target site of a user's body toward a device for promoting expression of bodily fluid from the target site, as set forth in step 1100.

At step 1200, the urging results in the sequential increasing of an area of contact and applied pressure between a pressure application mechanism of the device and the user's body in the vicinity of the target site and in a direction toward the target site, to thereby urge bodily fluid toward the target site. Subsequently, the target site is lanced while the device promotes the expression of bodily fluid from the lanced target site in the manner described above, as set forth in step 1300.

One skilled in the art will recognize that process 1000 can be accomplished using devices for promoting bodily fluid expression from a target site as described herein and that various details of such devices and their use as described herein can be employed in process 1000.

It should be understood that various alternatives to the embodiments of the invention described herein may be employed in practicing the invention. It is intended that the following claims define the scope of the invention and that methods and structures within the scope of these claims and their equivalents be covered thereby.

## Claims

1. A device for promoting bodily fluid expression from a target site of a user's body, the device comprising:
means for sequentially increasing an area of contact and applied pressure between a pressure application mechanism of the device and the user's body, in the vicinity of the target site and in a direction toward the target site, as the target site is urged toward the device, to urge bodily fluid toward the target site; and
means for lancing the target site while the device promotes the expression of bodily fluid from the lanced target site.

2. The device of claim 1, wherein the pressure application mechanism includes a plurality of ribs with semicircular contact areas configured for providing the sequentially increasing area of contact and applied pressure.

3. The device of claim 2, wherein at least one of the semicircular contact areas is adapted to remain stationary during the sequentially increasing step.

4. The device of claim 3, wherein at least one of the plurality of ribs is a biased rib.

5. The device of claim 3, wherein the applied pressure results in a force in the range of 15N to 20N.

6. The device of claim 3, wherein the target site is a user's fingertip and the semicircular contact areas have a contour that limits deformation of a user's finger during use of the device.

7. The device of claim 1, wherein the pressure application mechanism for urging bodily fluid toward the target site includes:
a biased cam pivotally attached to the base; and
a stationary leverage bar offset from the biased cam by a distance whereby a portion of the user's body can be inserted between the biased cam and the stationary leverage bar.

8. The device of claim 1, wherein the target site is on an underside of a user's fingertip and the area of sequentially increasing area of contact and applied pressure is on the underside of the user's finger.

9. The device of claim 9, wherein the area of contact and applied pressure sequentially increases from a position below a last knuckle of the user's finger to half way between the last knuckle and the fingertip.
